# EUROPÄISCHE PATENTANMELDUNG

(11) **EP 3 598 953 A1**
(43) Veröffentlichungstag der Anmeldung: **29.01.2020**
(21) Anmeldenummer: 18185132.0
(22) Anmeldetag: 24.07.2018
(51) Int. Cl.: A61C 1/08

(54) **BOHRSCHABLONE FÜR EINEN KIEFERCHIRURGISCHEN EINGRIFF**

(71) Anmelder: Danube Private University GmbH, 3500 Krems-Stein (AT)
(72) Erfinder: VON SEE, Constantin, 3500 Imbach/Krems (AT)
(74) Vertreter: Sonn & Partner Patentanwälte

(57) **Zusammenfassung**

Bohrschablone 1 für einen kieferchirurgischen Eingriff an einem Patienten, wobei die Bohrschablone 1 ein Zahnprovisorium 5, in dem eine Bohrführung 3 zur Führung eines Bohrers in das Kiefer des Patienten vorgesehen ist, und zumindest ein mit dem Zahnprovisorium 5 verbundenes Halteelement 4 zur vorübergehenden Befestigung der Bohrschablone 1 am Gebiss 2 des Patienten aufweist.

## Beschreibung

Die Erfindung betrifft eine Bohrschablone für einen kieferchirurgischen Eingriff an einem Patienten.

Bohrschablonen werden bei kieferchirurgischen Eingriffen zur Führung von Bohrern oder anderen zahnärztlichen Instrumenten eingesetzt. Zu diesem Zweck weisen Bohrschablonen üblicherweise Bohrführungen auf, durch welche der Bohrer an die zu behandelnde Stelle herangeführt und ein im durch die Bohrschablone vorgegebenen Winkel gesetztes Bohrloch erzeugt werden kann. In der Regel verhindert die Bohrschablone dabei nicht nur ein unbeabsichtigtes Versetzen des Bohrers während des Eingriffs, sondern gibt auch die genaue Position des Bohrloches im Kiefer eines Patienten vor.

Bohrschablonen sind insbesondere bei der Setzung von dentalen Implantaten von Bedeutung, da deren richtige Position ausschlaggebend für die ästhetische und mechanische Rehabilitation des Patienten ist. Da ein Implantat in sechs Freiheitsgraden in den Kiefer gesetzt werden kann, stellt die richtige Positionierung des dentalen Implantats bzw. des Bohrloches eine große Herausforderung dar. Insbesondere im Frontzahnbereich gewährleisten Bohrschablonen bessere ästhetische Ergebnisse im Vergleich zu Implantatsetzungen ohne Bohrschablone. Falsch oder schlecht positionierte Implantate können dagegen einen erheblichen technischen Aufwand erforderlich machen und mit zusätzlichen Kosten und vor allem Leiden des Patienten verbunden sein. Sie können auch die Mundhygiene am dentalen Implantat erschweren und zu Entzündungsreaktionen am Implantat führen, die letztendlich eine Entfernung des Implantats zur Folge haben. Daher stellen bereits die Planung und Erzeugung von Bohrschablonen wichtige Teile der Implantatsetzung dar. Die Implantatsetzung umfasst daher zumindest die Planung, den eigentlichen chirurgischen Eingriff (mit Bohren und Einsetzen der Implantate) und die anschließende Einheilphase.

Um die für das Einsetzen der dentalen Implantate notwendigen Bohrlöcher im Kiefer richtig setzen zu können, sodass sie später durch optimale Ausnutzung des vorhandenen Platzes hinreichend Halt bieten und insbesondere keine Nerven verletzen, werden Bohrschablonen üblicherweise nach vorheriger Vermessung des Kiefers bzw. des Gebisses des Patienten computerunterstützt entworfen und anschließend durch abtragende oder aufbauende Verfahren produziert.

Während der Einheilphase der Implantate wird das frisch gesetzte dentale Implantat üblicherweise durch ein Zahnprovisorium geschützt, welches in einem eigenen Herstellungsprozess ebenfalls computerunterstützt gestaltet und erzeugt werden kann. In der Regel erfüllt das Zahnprovisorium neben dem Schutz der einheilenden Implantate und der Verhinderung von Zahnwanderungen von benachbarten Zähnen auch noch ästhetische Zwecke und ermöglicht zumindest ein vorsichtiges Kauen während der Einheilphase.

Die bekannten Herstellungsprozesse zur Erzeugung der Bohrschablone und des Zahnprovisoriums sind sehr zeitaufwendig und mit hohen Kosten verbunden. Und obwohl im Stand der Technik ein hoher Aufwand zur Planung und Herstellung der Bohrschablone und des Zahnprovisoriums betrieben wird, passt das Zahnprovisorium zuweilen nicht exakt zum gesetzten dentalen Implantat. Es wäre daher wünschenswert, wenn das Zahnprovisorium genauer auf das gesetzte dentale Implantat abgestimmt und der Herstellungsaufwand minimiert werden könnte. Ebenso wäre es von Vorteil, wenn die Dauer bzw. Komplexität des zahnärztlichen Eingriffs zum Setzen der Implantate sowie des Zahnprovisoriums verringert werden könnte.

Der Erfindung liegt somit die Aufgabe zugrunde, eine Bohrschablone der eingangs genannten Art zur Verfügung zu stellen, die zumindest einzelne Nachteile des Standes der Technik reduziert bzw. zur Gänze zu beseitigt. Die Erfindung setzt sich insbesondere zum Ziel, die Passgenauigkeit zwischen dem Zahnprovisorium und dem dentalen Implantat zu erhöhen und gleichzeitig den Herstellungsaufwand des Zahnprovisoriums zu reduzieren, und/oder die Gesamtdauer bzw. Komplexität der zahnärztlichen Behandlung zu verringern.

Die gestellte Aufgabe wird dabei dadurch gelöst, dass die Bohrschablone ein Zahnprovisorium, in dem eine Bohrführung zur Führung eines Bohrers in das Kiefer des Patienten vorgesehen ist, und zumindest ein mit dem Zahnprovisorium verbundenes Halteelement zur vorübergehenden Befestigung der Bohrschablone am Gebiss des Patienten aufweist. Durch die erfindungsgemäße Kombination der Bohrschablone mit dem Zahnprovisorium können beide Elemente in einem gemeinsamen Herstellungsprozess geplant und erzeugt werden, wodurch der Herstellungsaufwand reduziert wird. Zudem ermöglicht die Kombination der beiden bisher voneinander getrennten, zu unterschiedlichen Zwecken eingesetzten und in verschiedenen Phasen der Implantatsetzung verwendeten Elemente die verbesserte Abstimmung des Zahnprovisoriums auf das Bohrloch und damit auf das dentale Implantat. Darüber hinaus wird die Dauer bzw. Komplexität des zahnärztlichen Eingriffs aufgrund des durch die Erfindung ermöglichten kombinierten Setzens der Implantate sowie des Zahnprovisoriums verringert.

Um während des Eingriffs den Bohrer in das Kiefer des Patienten zu führen, weist das Zahnprovisorium erfindungsgemäß eine Bohrführung auf, welche vorzugsweise als Kanal ausgeführt ist, der von einer ersten Seite des Zahnprovisoriums auf eine zweite Seite führt und das Zahnprovisorium durchdringt. Zweckmäßigerweise ist der Kanal gerade, d.h. krümmungsfrei, und bis auf zwei endseitige Öffnungen an den Seiten des Zahnprovisoriums geschlossen. Die Bohrführung kann nach der Bohrung mit einem geeigneten Material verschlossen werden, um die Ästhetik des verwendeten Zahnprovisoriums zu erhöhen. Damit das Zahnprovisorium bzw. die Bohrschablone während des kieferchirurgischen Eingriffs fixiert werden kann, ist das Zahnprovisorium erfindungsgemäß mit zumindest einem Halteelement verbunden. Das Halteelement verhindert das unbeabsichtigte Versetzen des Zahnprovisoriums bzw. der Bohrschablone und kann die Orientierung und Positionierung der Bohrschablone vorgeben. Vorzugsweise ist das Halteelement zu diesem Zweck an das Gebiss des Patienten angepasst. Falls es erforderlich ist, kann auch das Halteelement eine Bohrführung aufweisen.

Die Bohrschablone besteht vorzugsweise aus einem Kunststoff oder Keramik und kann insbesondere einstückig aus einem einzigen Material gefertigt sein. Es kann aber auch vorgesehen sein, dass die Bohrschablone verschiedene Materialien umfasst. Beispielsweise kann das Zahnprovisorium aus einem anderen Material wie das zumindest eine Halteelement bestehen.

Grundsätzlich kann bei Zahnprovisorien zwischen implantatgetragenen und nicht-implantatgetragenen Zahnprovisorien unterschieden werden. Implantatgetragene Zahnprovisorien sind bereits während der Einheilphase des dentalen Implantats mit diesem verbunden. Um das eingesetzte dentale Implantat zu schützen, soll daher bei der Planung von implantatgetragenen Implantaten darauf geachtet werden, dass Kaukontakt mit benachbarten Zähnen vermieden wird und ein Material für das Zahnprovisorium verwendet wird, das eine möglichst geringe Krafteinleitung in das dentale Implantat gewährleistet. Im Unterschied dazu sind nichtimplantatgetragene Zahnprovisorien während der Einheilphase nicht mit dem dentalen Implantat verbunden. Um das Zahnprovisorium dennoch fixieren zu können, werden im Stand der Technik Klebebrücken ("Marylandbrücken") oder provisorische Brücken zu benachbarten Zähnen verwendet. Daneben gibt es nichtimplantatgetragene Zahnprovisorien, die als Klammerprothesen ausgeführt sind und über einen Bügel am Gaumen oder an den benachbarten Zähnen befestigt sind. Das Zahnprovisorium, welches die erfindungsgemäße Bohrschablone aufweist, ist bevorzugt als implatantatgetragenes oder als nicht-implantatgetragenes Zahnprovisorium einsetzbar bzw. vorgesehen.

Aus ästhetischen und praktischen Gründen ist es vorteilhaft, wenn das zumindest eine Halteelement über eine lösbare Verbindung mit dem Zahnprovisorium verbunden ist. Demnach kann das Halteelement während der Behandlung des Patienten vom Zahnarzt einfach per Hand oder mit Hilfe eines entsprechenden Werkzeugs vom Zahnprovisorium gelöst werden. Überschüssige Teile können im Bedarfsfall auch mit einer Feile weggeschliffen werden. Das Zahnprovisorium kann anschließend an einer dafür vorgesehenen Stelle, insbesondere benachbart zum in das Kiefer des Patienten eingesetzten dentalen Implantat, platziert werden.

Zur leichteren Lösbarkeit ist in einer bevorzugten Ausführungsform vorgesehen, dass die lösbare Verbindung durch eine Materialverjüngung oder eine Perforation gebildet wird. Dadurch kann das Halteelement besonders einfach, beispielsweise durch mehrmaliges Verknicken, von dem Zahnprovisorium gelöst werden.

Um das Risiko eines unbeabsichtigten Versetzens des Bohrers während des Eingriffs weiter zu reduzieren, ist es günstig, wenn das zumindest eine Halteelement zum Aufsetzen auf einen oder mehrere Zähne des Gebisses des Patienten zur Positionierung der Bohrschablone am Gebiss des Patienten ausgeformt ist. Durch das Aufsetzen des Halteelements auf einen oder mehrere Zähne wird der Halt des Halteelements und damit der Bohrschablone erhöht.

In einer weiteren bevorzugten Ausführungsform weist das zumindest eine Halteelement eine Öffnung, insbesondere eine an einen oder mehrere Zähne des Gebisses des Patienten angepasste Öffnung, zur Positionierung der Bohrschablone am Gebiss des Patienten auf. Diese Öffnung kann unter anderem durch einen offenen Hohlraum oder eine Wölbung des Halteelements gebildet werden, die innenseitig, d.h. der im Gebrauchszustand dem Gebiss zugewandten Seite, ein Negativ eines oder mehrerer Zähne des Patienten darstellt. Durch diese Maßnahme wird die Bohrschablone vor Verdrehungen oder Versetzungen während des Eingriffs besonders gut gesichert und typischerweise eine exakte Soll-Positionierung vorgegeben.

Um das Zahnprovisorium während der Einheilphase nicht mit dem frisch gesetzten dentalen Implantat verbinden zu müssen, ist es günstig, wenn das Zahnprovisorium zumindest einen Haltesteg zur Verbindung mit einer Zahnoberfläche aufweist, wobei der Haltesteg vorzugsweise durch einen Verbindungsabschnitt des Zahnprovisoriums zu dem zumindest einen Halteelement gebildet wird. Damit kann der Haltesteg quasi als zahnärztliche Brücke zu anderen Zähnen fungieren, wobei die Brücke bei der Herstellung der Bohrschablone sozusagen als Teil der Bohrschablone miterzeugt wurde. Durch den Haltesteg ist das Zahnprovisorium besonders einfach als nicht-implantatgetragenes Zahnprovisorium einsetzbar. Der Haltesteg ist zweckmäßigerweise zur Verbindung mit natürlichen, zum dentalen Implantat benachbarten Zähnen geeignet und wird bevorzugt durch vom Zahnprovisorium auskragende Verbindungselemente wie beispielsweise Flügel, Klammern oder Kappen gebildet. Der Haltesteg besteht zudem vorzugsweise aus im Wesentlichen festem, d.h. unflexiblem Material. Insbesondere kann die gesamte Bohrschablone aus dem gleichen Material bestehen. Während der zahnärztlichen Behandlung zur Platzierung des Zahnprovisoriums kann der Haltesteg mit einem geeigneten Klebstoff oder Befestigungskunststoff versehen und mit der Zahnoberfläche benachbarter Zähne verbunden werden. Falls erforderlich kann zuvor die Zahnoberfläche der benachbarten Zähne, insbesondere deren Schmelzoberfläche, säuregeätzt werden. Zur leichteren Herstellung kann der Haltesteg durch einen Verbindungsabschnitt zwischen dem Zahnprovisorium und dem Halteelement gebildet werden, welcher insbesondere an einem dem Halteelement zugewandten Ende über eine lösbare Verbindung verfügt.

Damit implantatgetragene Zahnprovisorien, d.h. Zahnprovisorien, die mit dem eingesetzten Implantat bereits während der Einheilphase verbunden sind, am Implantat befestigt werden können, ist es günstig, wenn das Zahnprovisorium an einer im eingesetzten Zustand dem Kiefer des Patienten zugewandten Seite eine Anschlussstelle zur mittelbaren oder unmittelbaren Verbindung mit einem Implantat aufweist. Vorteilhafterweise kann die Anschlussstelle an das Implantat angepasst sein und ein Gewinde aufweisen, mit dem das Zahnprovisorium mit dem Implantat verbunden werden kann. Vorzugsweise ermöglicht die Anschlussstelle im mit dem Implantat verbundenen Zustand eine zumindest teilweise Drehung des Zahnprovisoriums relativ zum Implantat, um nachträgliche Anpassungen des Zahnprovisoriums in Bezug auf die Position relativ zum Gebiss des Patienten zu ermöglichen. Zwischen dem Implantat und der Anschlussstelle kann im verbundenen Zustand auch noch ein Zwischenstück als Adapter angeordnet sein, wobei in diesem Fall die Anschlussstelle an das Zwischenstück angepasst ist. Es ist natürlich auch denkbar, das Zahnprovisorium gleichzeitig als implantatgetragenes bzw. nicht-implantatgetragenes vorzusehen, d.h. sowohl mit Anschlussstelle, als auch mit Haltestegen zu versehen, wodurch der Zahnarzt beispielsweise abhängig von der Mundsituation des Patienten während der Behandlung wählen kann, ob er das Zahnprovisorium implantatgetragen bzw. nicht-implantatgetragen ausführt.

In einer bevorzugten Ausführungsform ist vorgesehen, dass in die Bohrführung eine vorzugsweise metallische Hülse eingefügt ist. Die Hülse dient zur Führung des Bohrers und verhindert Beschädigungen des Zahnprovisoriums durch Bohrabrieb. Die Hülse kann entnehmbar sein. Wenn es zweckmäßig ist, kann die Hülse auch aus dem Zahnprovisorium hervorstehen.

Um ein zu tiefes Eindringen des Bohrers zu verhindern, können in einem Randbereich der Bohrführung entfernbare Anschlagelemente zur Bohrtiefenbegrenzung des Bohrers vorgesehen sein. Diese sind vorteilhafterweise über eine lösbare Verbindung mit der Bohrschablone, insbesondere mit dem Zahnprovisorium, verbunden.

Aus ästhetischen Gründen ist es günstig, wenn zumindest eine Oberfläche des Zahnprovisoriums aus opaken, vorzugsweise weißem bzw. zahnfarbenem, Material ist. Insbesondere wird die Herstellung der Bohrschablone vereinfacht, wenn das gesamte Zahnprovisorium oder gar die gesamte Bohrschablone aus dem gleichen opaken Material besteht.

Um während des Eingriffs mehrere Werkzeuge gleichzeitig einsetzen zu können, ist es vorteilhaft, wenn das Zahnprovisorium eine Arretierung für ein Werkzeug wie beispielsweise einen Löffel aufweist. Löffel sind in diesem Zusammenhang zahnärztliche Werkzeuge, die den Innendurchmesser der Bohrführung reduzieren, sodass auch verschiedene Bohrer wie Zentrierbohrer, Pilotbohrer, Tiefenbohrer, Kopfsenker und Gewindebohrer mit jeweils unterschiedlichen Durchmessern präzise geführt werden können. Erfindungsgemäß können diese Werkzeuge mit der Arretierung verbunden und dadurch verrastet werden. Zu diesem Zweck kann die Arretierung als Bajonettverschluss ausgeführt sein.

Die Erfindung wird nachstehend an Hand von bevorzugten Ausführungsformen, auf die sie allerdings nicht eingeschränkt ist, näher erläutert.
Fig. 1 zeigt eine Bohrschablone, wie sie aus dem Stand der Technik bekannt ist.
Fig. 2 zeigt eine erfindungsgemäße Bohrschablone auf einem Gebiss eines Patienten in einer ersten Ausführungsform.
Fig. 3 zeigt die erfindungsgemäße Bohrschablone der ersten Ausführungsform in einer anderen Ansicht.
Fig. 4 zeigt eine erfindungsgemäße Bohrschablone auf einem Gebiss eines Patienten in einer zweiten Ausführungsform.

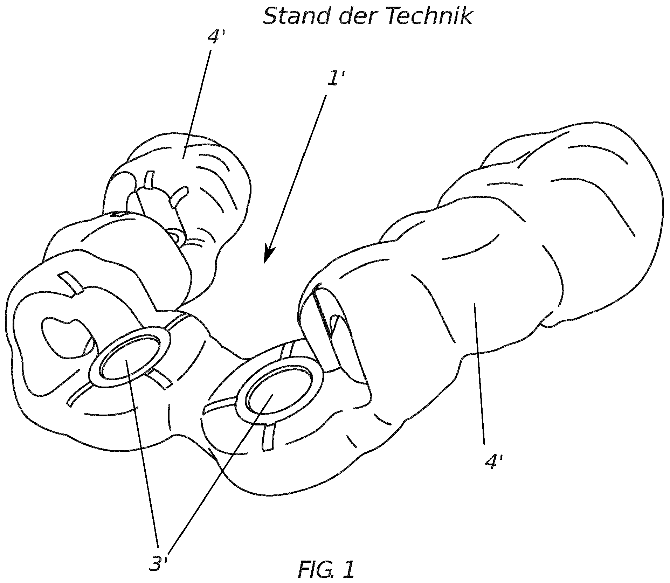

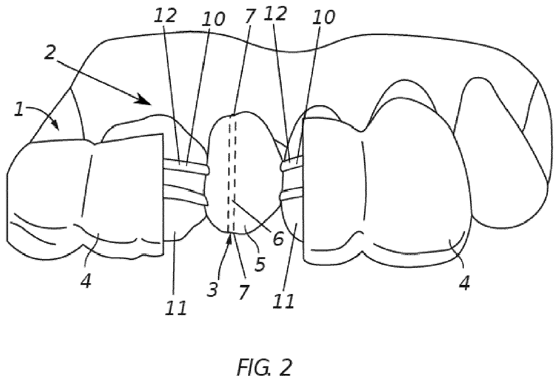

Fig. 1 zeigt zum Vergleich eine bereits aus dem Stand der Technik bekannte Bohrschablone 1' mit mehreren Durchgangslöchern als Bohrführungen 3'. Zur Fixierung der Bohrschablone 1' an einem Gebiss eines Patienten (nicht gezeigt) verfügt die Bohrschablone über Halteelemente 4', die an die Zähne des Patienten angepasst sind. Während eines kieferchirurgischen Eingriffs wird die Bohrschablone 1' auf das Gebiss aufgesetzt und ein Bohrer durch die Bohrführung 3' geführt, um die Bohrlöcher für die dentalen Implantate zu setzen. Nach dem Eingriff wird die Bohrschablone 1' entfernt und die gesetzten Implantate mit einem eigens gefertigten Zahnprovisorium (nicht gezeigt) geschützt. Bohrschablonen aus dem Stand der Technik verfügen im Gegensatz zur erfindungsgemäßen Bohrschablone (vgl. Fig. 2 bis 4) nicht über ein integriertes Zahnprovisorium und können daher nach der Bohrung zur Gänze entsorgt werden, da sie keiner weiteren Verwendung mehr zugeführt werden können.
Fig. 2 zeigt im Vergleich dazu die erfindungsgemäße Bohrschablone 1 in einer ersten Ausführungsform, welche in der gezeigten Darstellung auf einem Gebiss 2 eines Patienten platziert ist. Die Bohrschablone 1 verfügt zur Fixierung vorteilhafterweise über zwei Halteelemente 4, zwischen denen ein Zahnprovisorium 5 angeordnet ist. In Fig. 2 ist das Zahnprovisorium 5 zwischen zwei natürlichen Zähnen des Gebisses 2 eingefügt. Um den Bohrer während des Eingriffs an den Kiefer des Patienten zu führen, weist das Zahnprovisorium 5 eine Bohrführung 3 auf. Vorzugsweise ist die Bohrführung 3 als Kanal 6 ausgeführt, der von einer ersten Seite des Zahnprovisoriums 5 auf eine gegenüberliegende zweite Seite führt und das Zahnprovisorium 5 durchdringt. Zweckmäßigerweise ist der Kanal 6 gerade, d.h. krümmungsfrei, und bis auf zwei endseitige Öffnungen 7 an den gegenüberliegenden Seiten des Zahnprovisoriums 5 geschlossen. Durch die strichlierten Linien ist angedeutet, dass die Bohrführung 3 im Wesentlichen innerhalb des Zahnprovisoriums 5 verläuft und nur durch die endseitigen Öffnungen 7 zugänglich ist.

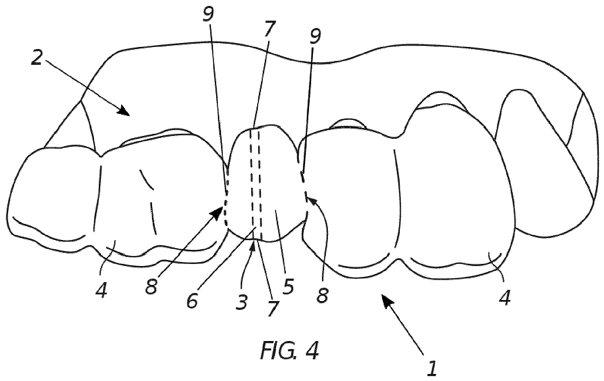

Nach der Bohrung kann die Bohrschablone 1 vom Gebiss des Patienten genommen und das Zahnprovisorium 5 von Hand durch mehrmahliges Verknicken oder mit Hilfe eines entsprechenden Werkzeugs von den Halteelementen 4 gelöst werden. Zur leichteren Lösbarkeit kann das Zahnprovisorium 5 über eine lösbare Verbindung mit den Halteelementen 4 verbunden sein. Insbesondere ist es hierzu günstig, wenn die lösbare Verbindung durch eine Materialverjüngung oder eine Perforation gebildet wird (vgl. Fig. 4). Nach dem Lösen des Zahnprovisoriums 5 von den Halteelementen 4 kann dieses an der dafür vorgesehenen Stelle im Gebiss 2 des Patienten platziert werden. Dies wird regelmäßig jene Stelle sein, an der das Zahnprovisorium 5 während des Eingriffs zur Führung des Bohrers platziert war. Aus ästhetischen Gründen kann zumindest eine Seite des Zahnprovisoriums 5 aus zahnfarbenem Material hergestellt sein. Zur Vereinfachung der Herstellung kann die ganze Bohrschablone 1 aus dem gleichen zahnfarbenen Material gefertigt sein.

Zur Befestigung des Zahnprovisoriums 5 am Gebiss 2 des Patienten nach Entfernung der Halteelemente kann das Zahnprovisorium 5, wie im gezeigten Ausführungsbeispiel, zumindest einen Haltesteg 10 zur Verbindung mit einer Zahnoberfläche 11 aufweisen, wobei der Haltesteg 10 vorzugsweise durch einen Verbindungsabschnitt 12 des Zahnprovisoriums 5 zu einem Halteelement 4 gebildet wird. Das Zahnprovisorium der ersten Ausführungsform eignet sich daher besonders als nicht-implantatgetragenes Zahnprovisorium. Die lösbare Verbindung zwischen dem Zahnprovisorium 5 und einem Halteelement 4 ist vorzugsweise an dem entsprechenden Halteelement 4 zugeordneten Ende des Haltestegs 10 angeordnet.

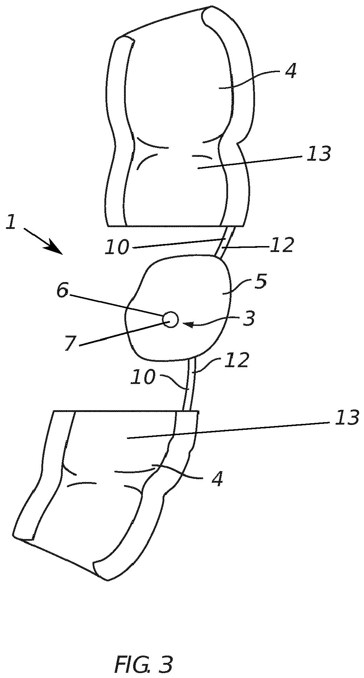

Zur verbesserten Fixierung der Bohrschablone 1 während des Eingriffs ist es günstig, wenn die Halteelemente 4 zum Aufsetzen auf einen oder mehrerer Zähne zur Positionierung der Bohrschablone 1 am Gebiss 2 des Patienten ausgeformt sind. Dies ist insbesondere aus Fig. 3 ersichtlich, welche die Bohrschablone 1 in einer Ansicht von unten, d.h. mit Blick auf die im Gebrauchszustand dem Gebiss 2 des Patienten zugewandte Seite, zeigt. Insbesondere kann das zumindest eine Halteelement 4 eine Öffnung 13, vorzugsweise eine an einen oder mehrere Zähne des Gebisses 2 des Patienten angepasste Öffnung 13, zur Positionierung der Bohrschablone 1 am Gebiss 2 des Patienten aufweisen. Diese Öffnung 13 kann unter anderem durch einen offenen Hohlraum oder eine Wölbung des Halteelements 4 gebildet werden, die innenseitig, d.h. der im Gebrauchszustand dem Gebiss zugewandten Seite, ein Negativ eines oder mehrerer Zähne des Patienten darstellt.

In Fig. 4 ist eine zweite Ausführungsform der Bohrschablone 1 dargestellt. Wie die erste Ausführungsform ist das Zahnprovisorium 5 zwischen zwei Halteelementen 4 eingefügt und weist über eine als Kanal 6 ausgeführte Bohrführung 3 auf. An Stelle der oben im Zusammenhang mit Fig. 2 beschriebenen Verbindungsabschnitte 12 bzw. Haltestege 10 ist das Zahnprovisorium 5 direkt über eine lösbare Verbindung 8 mit den Halteelementen 4 verbunden, welche unter anderem durch eine Materialverjüngung oder eine Perforation 9 gebildet werden kann.

Das Zahnprovisorium 5 der zweiten Ausführungsform ist insbesondere für den Einsatz als implantatgetragenes Zahnprovisorium 5 geeignet. Zu diesem Zweck kann das Zahnprovisorium 5 an einer im eingesetzten Zustand dem Kiefer des Patienten zugewandten Seite eine an das Implantat angepasste Anschlussstelle (nicht gezeigt) zur mittelbaren oder unmittelbaren Verbindung mit einem Implantat aufweisen. Vorteilhafterweise kann die Anschlussstelle ein Gewinde aufweisen, mit dem das Zahnprovisorium 5 mit dem Implantat verbunden werden kann. Vorzugsweise ist die Anschlussstelle dabei derart ausgeführt, dass das Zahnprovisorium 5 im verbundenen Zustand mit dem Implantat zumindest teilweise drehbar zum Implantat ist, um nachträgliche Anpassungen des Zahnprovisoriums 5 hinsichtlich seiner Position zu ermöglichen. Zwischen der Anschlussstelle und dem Implantat kann auch ein Zwischenstück als Adapter vorgesehen sein, wobei in diesem Fall die Anschlussstelle an das Zwischenstück angepasst ist.

## Patentansprüche

1. Bohrschablone (1) für einen kieferchirurgischen Eingriff an einem Patienten, **dadurch gekennzeichnet, dass** die Bohrschablone (1) ein Zahnprovisorium (5), in dem eine Bohrführung (3) zur Führung eines Bohrers in das Kiefer des Patienten vorgesehen ist, und zumindest ein mit dem Zahnprovisorium (5) verbundenes Halteelement (4) zur vorübergehenden Befestigung der Bohrschablone (1) am Gebiss (2) des Patienten aufweist.

2. Bohrschablone (1) nach Anspruch 1, **dadurch gekennzeichnet, dass** das zumindest eine Halteelement (4) über eine lösbare Verbindung (8) mit dem Zahnprovisorium (5) verbunden ist.

3. Bohrschablone (1) nach Anspruch 2, **dadurch gekennzeichnet, dass** die lösbare Verbindung (8) durch eine Materialverjüngung oder eine Perforation (9) gebildet wird.

4. Bohrschablone (1) nach einem der Ansprüche 1 bis 3, **dadurch gekennzeichnet, dass** das zumindest eine Halteelement (4) zum Aufsetzen auf einen oder mehrerer Zähne des Gebisses (2) des Patienten zur Positionierung der Bohrschablone (1) am Gebiss (2) des Patienten ausgeformt ist.

5. Bohrschablone (1) nach Anspruch 4, **dadurch gekennzeichnet, dass** das zumindest eine Halteelement (4) eine Öffnung (13), insbesondere eine an einen oder mehrere Zähne des Gebisses (2) des Patienten angepasste Öffnung, zur Positionierung der Bohrschablone (1) am Gebiss (2) des Patienten aufweist.

6. Bohrschablone (1) nach einem der Ansprüche 1 bis 5, **dadurch gekennzeichnet, dass** das Zahnprovisorium (5) zumindest einen Haltesteg (10) zur Verbindung mit einer Zahnoberfläche (11) aufweist, wobei der Haltesteg (10) vorzugsweise durch einen Verbindungsabschnitt (12) des Zahnprovisoriums (5) zu dem zumindest einen Halteelement (4) gebildet wird.

7. Bohrschablone (1) nach einem der Ansprüche 1 bis 6, **dadurch gekennzeichnet, dass** das Zahnprovisorium (5) an einer im eingesetzten Zustand dem Kiefer des Patienten zugewandten Seite eine Anschlussstelle zur mittelbaren oder unmittelbaren Verbindung mit einem Implantat aufweist.

8. Bohrschablone (1) nach einem der Ansprüche 1 bis 7, **dadurch gekennzeichnet, dass** in die Bohrführung (3) eine vorzugsweise metallische Hülse eingefügt ist.

9. Bohrschablone (1) nach einem der Ansprüche 1 bis 8, **dadurch gekennzeichnet, dass** in einem Randbereich der Bohrführung (3) entfernbare Anschlagelemente zur Bohrtiefenbegrenzung des Bohrers vorgesehen sind.

10. Bohrschablone (1) nach einem der Ansprüche 1 bis 9, **dadurch gekennzeichnet, dass** zumindest eine Oberfläche des Zahnprovisoriums (5) aus opaken, vorzugsweise zahnfarbenem, Material ist.

11. Bohrschablone (1) nach einem der Ansprüche 1 bis 10, **dadurch gekennzeichnet, dass** das Zahnprovisorium (5) eine Arretierung für ein Werkzeug wie beispielsweise einen Löffel aufweist.
